(19) Europäisches Patentamt
European Patent Office
Office européen des brevets

(11) **EP 3 824 804 A1**

(12) **EUROPEAN PATENT APPLICATION**

(43) Date of publication:
26.05.2021 Bulletin 2021/21

(51) Int Cl.:
*A61B 5/05* (2021.01)     *A61B 5/00* (2006.01)
*A61B 5/024* (2006.01)     *A61B 5/08* (2006.01)

(21) Application number: 19211100.3

(22) Date of filing: 25.11.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(71) Applicant: **Koninklijke Philips N.V.**
**5656 AG Eindhoven (NL)**

(72) Inventors:
• **DOODEMAN, Gerardus Johannes Nicolaas**
**5656 AE Eindhoven (NL)**

• **BLOM, Antonius Hermanus Maria**
**5656 AE Eindhoven (NL)**
• **PEETERS, Wouter Herman**
**5656 AE Eindhoven (NL)**
• **STEUNEBRINK, Tim Patrick**
**5656 AE Eindhoven (NL)**
• **VERNOOIJ, Carlijn Andrea**
**5656 AE Eindhoven (NL)**

(74) Representative: **Philips Intellectual Property &**
**Standards**
**High Tech Campus 5**
**5656 AE Eindhoven (NL)**

(54) **INDUCTIVE SENSING SYSTEM AND METHOD**

(57)     An inductive sensing system (8) has a resonator circuit (10) with an antenna (12) for simultaneously applying electromagnetic signals to a body and sensing secondary electromagnetic signals returned from the body. The system includes signal sensing means (30) which is configured to detect a measure indicative of an imaginary part of an additional inductance component added to the resonator circuit by the secondary electromagnetic signals but which does not measure the real part. In particular, the signal sensing means may be configured to detect a measure indicative of damping in the resonator circuit (e.g. a damping factor), and comprises no means for detecting any measure indicative of variations in a natural frequency of the resonator circuit.

FIG. 2

EP 3 824 804 A1

**Description**

FIELD OF THE INVENTION

This invention relates to an inductive sensing system and method, in particular for sensing electromagnetic signals emitted from a body responsive to application to the body of electromagnetic excitation signals.

BACKGROUND OF THE INVENTION

Inductive sensing can be used as a means of non-invasive investigation of properties of a body.

In one advantageous area of application, inductive sensing can be used as a means of non-invasively investigating physiological characteristics, in particular heart and lung dynamics. For instance it can be used to measure respiration rate, respiration depth and pulse rate. It can more broadly measure mechanical movements and dynamical changes of internal bodily structures such as the heart, the lungs, or arteries. For instance, it may be used to sense the cyclically varying internal volume or dimensions of chambers of the heart, or of the lungs, or of mechanical activity of arteries, e.g. changing arterial volumes over heart cycles.

Inductive sensing is based on generation of a primary alternating magnetic field via a primary antenna loop, which leads to the induction of eddy currents and a consequent secondary magnetic field in conductive material or tissue within the primary magnetic field. Interaction of the secondary magnetic field with the primary loop or the primary magnetic field can be used to detect patterns of movement within probed bodies, in particular those comprising a water content.

In particular, this field interaction leads to changes in the detectable electrical characteristics of the current running through the antenna coil. For example, the current frequency can be changed, and/or the current amplitude can be dampened.

Of particular advantage is use of inductive sensing for detecting physiological signals, (otherwise known as kymographic signals) such as heartbeats and breathing patterns.

In inductive sensing, a signal generator (such as an oscillator) is connected to a loop antenna. The oscillator is an amplifier, typically consisting of one or more transistors, which induces a resonant state in a coupled circuit, in combination with an inductance source and capacitance source. The inductance is provided by the loop antenna, while the capacitance is provided by an optional capacitor component placed in parallel to the loop, together with parasitic capacitances of the loop with itself and its environment, and the oscillator parasitic capacitances. The total system is called the resonator.

The secondary magnetic field generated by the body has the effect at the antenna loop of adding an additional complex inductance to the resonator circuit, which thereby leads to detectable changes in the circuit current. The real part of the additional inductance is detectable as changes in frequency of the oscillator circuit current. The imaginary part of the additional inductance is detectable as changes in amplitude of the oscillator circuit current (or voltage).

Inductive sensing carries the disadvantage that it can be power intensive to operate due to the need to continuously drive the resonator circuit with the oscillating drive signal. This limits potential applications for the technology. For example, it is difficult to apply inductive sensing within a portable or wireless form factor, since the battery drain is typically too rapid to allow application for more than a relatively short period.

Improvements would therefore be of advantage which permit application of the inductive sensing device with reduced power consumption, for example to enable use with a battery power source for longer periods of use.

SUMMARY OF THE INVENTION

The invention is defined by the claims.

According to examples in accordance with an aspect of the invention, there is provided an inductive sensing system for sensing electromagnetic signals returned from a body responsive to application of electromagnetic excitation signals to said body, the system comprising:

a resonator circuit comprising: a loop antenna and an electronic signal generator coupled to the antenna, for driving the antenna with a drive signal to cause it to generate the electromagnetic excitation signals, the resonator circuit having a resonance frequency,
a signal sensing means, arranged for sensing, simultaneously with signal generation, a measure indicative of a damping exhibited by the resonator circuit, for example relative to the drive signal supplied to the resonator circuit,
wherein the inductive sensing system is adapted to measure only said damping in the resonator circuit, and is not adapted (or comprises no means) to detect any measure indicative of variation in frequency in the resonator circuit, e.g. variation in natural frequency as caused by interaction of the secondary electromagnetic signals returned from the body.

The frequency refers for example to instantaneous natural frequency of the resonator circuit, i.e. the natural frequency of oscillations in the resonator circuit at a given time, i.e. the frequency at which the resonator would naturally oscillate or is oscillating. The damping may refer to a damping factor of the resonator circuit.

Oscillations in the resonator at a given time may in general have a different amplitude or frequency to the drive signal with which the resonator circuit is being forced. These oscillations define an instantaneous measurable signal in the resonator circuit.

It has been recognized by the inventors that one source of significant power consumption is in the signal sensing components of the system. In previous inductive sensing devices these have been adapted to measure at least the real (and possible also imaginary) part of an additional inductance component induced at the resonator circuit antenna by the secondary electromagnetic signals returned from the body. These real and imaginary parts manifest as changes in the natural frequency and the natural damping factor respectively of the resonator circuit. Thus previous devices have included circuitry to measure at least natural frequency variations (real part of additional inductance) and sometimes also circuit damping factor variations (imaginary part of additional inductance) in the resonator circuit when the device is applied to a body. Damping of the circuit can be measured via amplitude variations in the resonator circuit oscillations for example.

It has previously been thought that both real and imaginary parts are needed for maximum accuracy or precision in signals extraction from the body. For example, the particular frequency can provide an indication of depth from which signals are originating in the body.

The recognition of the inventors for the present invention is that sensing the real part of the additional inductance component (e.g. via variations in natural frequency of the oscillator circuit) consumes significantly more power than sensing the imaginary part (e.g. via amplitude variations in the oscillator signals). It has also been recognized that for simple applications, e.g. where only one or two particular types of signals are required to be extracted at a time from the body, measurement of just the imaginary part of the additional inductance component (via damping in the resonator circuit) is sufficient. This allows significant power consumption savings to be made, although at the slight cost of reduced versatility or precision in the sensing device. It hence provides a 'slim-line' version of the device, configured in particular for low power applications, e.g. sensing devices for use with battery power, e.g. mobile sensing systems.

In addition, measuring frequency requires more complex, and thus expensive, measurement apparatus or circuitry than does measurement of damping and hence the system is simplified, and manufacture costs reduced, by only extracting a measure indicative of damping.

Sensing damping may mean sensing variations in a damping factor of the resonator circuit. This may in some cases mean sensing damping as exhibited in an instantaneous measurable signal or current in the resonator circuit. The instantaneous measurable signal in this case is a resultant electrical signal or current caused by the interaction of the drive signal and the returned electromagnetic signals from the body. The measurable signal is a measurable current or voltage in the resonator circuit (e.g. as a function of time) for example. The measurable signal may mean an instantaneous or real time signal or current running through the resonator circuit, e.g. the antenna, i.e. an operating current.

Damping might also be sensed in other examples in a more indirect way, for example via monitoring variation of an amplification factor required to be applied by an amplifier in order to keep a power level of an extracted signal constant. This will be explained in more detail to follow.

Preferably the antenna is a loop antenna comprising a single winding

In some examples, the signal sensing means may be adapted to monitor variation in said damping over time. The signal sensing means may extract or derive a signal representative of damping as a function of time over some measurement or observation period.

In some examples, the signal sensing means may comprise a circuit arrangement electrically coupled with the resonator circuit. The circuit arrangement for example is arranged for processing or operating on an instantaneous operating signal oscillating in the oscillator circuit.

The signal sensing means may be adapted to detect a measure indicative of variations in an amplitude of a measurable signal of the resonator circuit, i.e. variations in an amplitude of oscillations in the resonator circuit as a function of time.

In this example, sensing of the damping comprises sensing variations in an amplitude or natural amplitude of the measurable signal in the resonator circuit, i.e. the oscillations in the resonator circuit. This means for example sensing and monitoring variations in an amplitude of the current or voltage for example in the resonator circuit over time. It may alternatively comprise monitoring variations in an amplification applied to output signals extracted from the resonator circuit in order to keep them at a constant power level.

In some examples, the sensing of the damping comprises deriving a measure indicative of a variation in the amplitude of the measurable resonator circuit signal compared with an amplitude of the drive signal.

Damping means effectively a suppression or reduction in the energy or power in the resonator circuit oscillations. Hence this can be sensed based on detecting change in the instantaneous amplitude of the resonator circuit running current (or voltage) relative to the drive signal with which it is being forced.

Amplitude may refer to voltage amplitude or current amplitude for example.

According to one or more sets of embodiments, a circuit arrangement comprised by the signal sensing means may comprise:

an amplitude measurement element arranged for extracting a signal indicative of amplitude of the resonator circuit signal;

a low pass or band pass filter arranged for filtering the extracted amplitude signal to reduce noise; and

an amplifier arranged to amplify the filtered amplitude signal.

The amplitude measurement element may comprise a rectifying diode. The signal indicative of amplitude of the resonator circuit signal may be indicative of the resonator circuit current or voltage amplitude for example.

The filter is for reducing high frequency noise, meaning noise of a frequency higher than typical rate of change of the signal amplitude for example.

In particular examples, the circuit arrangement may further comprise a DC offset adjustment element between the filter and the amplifier, arranged for adjusting any negative DC offset to a positive DC offset in advance of the amplitude signal passing to the amplifier.

DC offset means the (DC) bias or baseline of the signal.

Many types of common amplifier (and analog-to-digital converters) are able to handle only signals having a positive DC bias or offset voltage. Thus adding a DC offset adjustment element ensures all types of amplifier (and ADC) can be used.

The circuit arrangement preferably further comprises an analog to digital converter (ADC) arranged to receive the amplitude signal after amplification by the amplifier.

The system may further include a processor, e.g. a microprocessor, for receiving the digital converted amplitude signal for storage, subsequent communication to another component or device or system, or for performing signal processing to derive one or more physiological parameters for example.

Additionally or alternatively to the use of a circuit arrangement, the signal sensing means may in some examples comprise a magnetic field sensor arranged in use to sense a magnetic field (e.g. magnetic field strength) to which the antenna of the resonator circuit is exposed. The magnetic field sensor is arranged to sense the magnetic field at the location of the antenna loop for example. It is arranged to sense the magnetic field with which the antenna is magnetically or inductively coupled for example, or the magnetic field by which the antenna is being electrically influenced in terms of its instantaneous oscillations.

The field strength of the magnetic field with which the loop antenna is coupled provides an indirect measure of variations in the damping factor. For example, an amplitude of magnetic field strength oscillations provide an indirect measure of consequent amplitude of the resonator oscillations, and thus of damping. Thus for example, variations downward in magnetic field strength oscillation amplitude indicates increased damping, and vice versa.

In some examples, the magnetic field sensor may be arranged to sense a magnetic field at a location radially inside of the loop of the loop antenna, e.g. at the radial center of the loop.

In accordance with one or more embodiments, the system may include signal processing means configured, based on sensed variations in damping in the resonator circuit as a function of time, to determine a respective measure indicative of one or more of heart rate and respiration rate.

In preferred examples, the signal processing means is configured to determine a measure indicative of respiration or breathing depth.

In accordance with one or more embodiments, the system may include control means configured in use to set a drive frequency of said drive signal, based on a target measurement depth within the body. The control means may comprise a controller or processor, for example a microcontroller or microprocessor.

The target measurement depth is a defined measurement depth corresponding to a desired depth of measurement within the body relative to a body surface (i.e. desired by a user or operator). Different anatomical features are located at different depths beneath the skin.

At a fixed drive signal frequency, the strength of the real and imaginary parts of the additional inductance component induced at the antenna by secondary electromagnetic signals emitted from the body will vary as a function of the depth in the body from which the signals are being emitted (i.e. the depth of the eddy currents). In other words, for a fixed depth in the body, the strength of the real and imaginary parts will each vary between a minimum and maximum value as a function of drive signal frequency. Thus, for a known desired measurement depth, it is possible to select the drive signal frequency so as to maximize measurement signal strength for the imaginary part (i.e. associated with damping) for that depth.

The control means may include for instance a memory storing a lookup table, the lookup table storing a plurality of target measurement depths and associated drive signal frequencies for maximizing strength of the measurement signal from the body

In some examples, the control means may be configured for receiving a user input indicative of the target measurement depth.

The system may include a user interface. Alternatively, the control means may be adapted in use to be communicatively coupleable with a user interface. The user interface may be a dedicated user interface such as a control panel, or may be provided for instance by a mobile communication device having a dedicated app installed for configuring the mobile device for communicating with the control means.

Examples in accordance with a further aspect of the invention provide a method for sensing electromagnetic signals returned from a body responsive to application of electromagnetic excitation signals to said body based on use of a resonator circuit comprising a loop antenna, the method comprising:

driving the loop antenna with a drive signal to cause it to generate the electromagnetic excitation signals, and sensing, simultaneously with signal generation, a damping exhibited in the resonator circuit, and wherein the method comprises sensing only said damping in the resonator circuit, and does not comprise detecting any measure indicative of a frequency or natural frequency of the resonator circuit.

In some examples, sensing of the damping comprises:

detecting a measure indicative of changes in the amplitude of a measurable resonator circuit signal compared with an amplitude of the drive signal; and/or
sensing variations in a magnetic field at the location of the antenna loop.

In accordance with one or more embodiments, the method may further comprise setting a frequency of the drive signal based on a target measurement depth within the body. This feature has already been explained above in relation to the system aspect of the invention.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 schematically illustrates eddy current induction within a body to which the system is applied;
Fig. 2 shows a schematic block diagram of components of an example system in accordance with one or more embodiments;
Fig. 3 shows a further schematic block diagram of components of an example system in accordance with one or more embodiments; and
Figs. 4 and 5 each show schematic bock diagrams of components of example signal conditioning blocks for use within sensing systems according to one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an inductive sensing system having a resonator circuit with an antenna for simultaneously applying electromagnetic signals to a body and sensing secondary electromagnetic signals returned from the body. The system includes signal sensing means which is configured to detect a measure indicative of an imaginary part of an additional inductance component added to the resonator circuit by the secondary electromagnetic signals but which does not measure the real part. In particular, the signal sensing means may be configured to detect a measure indicative of damping in the resonator circuit (e.g. a damping factor), and comprises no means for detecting any measure indicative of variations in a natural frequency of the resonator circuit.

Circuit components for measuring variations in frequency of the resonator oscillations (or natural frequency of the resonator circuit) are complex, expensive and consume a relatively high level of power. Thus by including only components for detecting variations in damping (e.g. via variations in amplitude), complexity of the system and also power consumption is reduced.

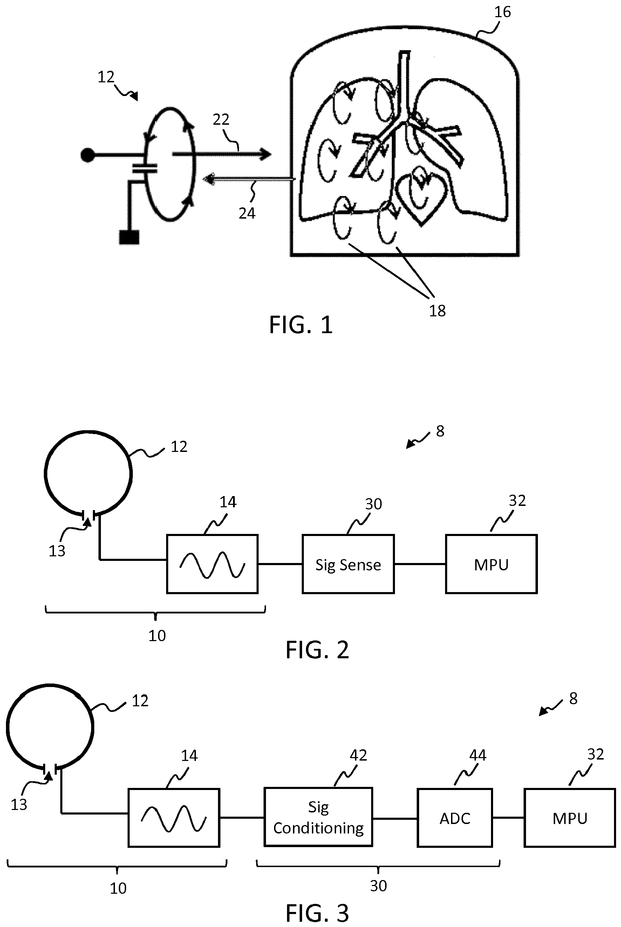

As discussed, embodiments of the invention make use principles of magnetic induction for sensing parameters of a body. Inductive sensing is based on generation of a primary alternating magnetic field via a primary antenna loop, which leads to the induction of eddy currents and a consequent secondary magnetic field in conductive material or tissue within the primary magnetic field. This is schematically illustrated in Fig. 1. A loop antenna 12 of a resonator circuit is driven with an alternating current (drive signal). This causes current oscillations in the antenna.

In use, the antenna 12 is brought into proximity with a body 16 to be probed. The driving of the antenna generates primary electromagnetic (EM) signals 22 which couple with the body and generate eddy currents 18 in the body. These eddy currents depend on the conductivity of the body. The eddy currents generate a secondary magnetic field 24. This secondary field interacts with the primary field 22 to alter the oscillation characteristics of the resonator circuit.

In particular, in general, when a loop antenna is brought into proximity with a body, the inductance, L, acquires an additional reflected inductance component, $L_r$, arising due to the eddy currents 18 induced in the stimulated body as a result of application of the excitation signals 22.

These eddy currents 18 in turn effectively make a contribution to the inductance of the loop antenna 12, due to the generation of a secondary time-varying magnetic flux. These eddy-current fluxes combine with the primary flux of the antenna, resulting in a greater induced back-EMF in the antenna, and hence a larger measurable effective inductance.

The added component of inductance arising from the eddy currents may be referred to synonymously in this disclosure as 'reflected inductance'.

In general, the reflected inductance, $L_r$, is complex, and can be expressed as

$$L_r = L_r' + i L_r'' \qquad (1)$$

where $L_r'$ is related to a reactive impedance of the antenna and $L_r''$ is related to resistive impedance of the antenna.

The addition of the reflected component of inductance $L_r$ leads to a detuning of the characteristics of the antenna (or resonator circuit). In particular, both the natural radial frequency of the coil antenna circuit and the damping factor of the coil antenna circuit change.

In particular, the real part of the additional inductance component, $L_r$, manifests in the natural frequency of the resonator circuit or antenna. The imaginary part of the additional inductance component manifests in the (natural) amplitude of oscillations of the resonator circuit.

In previously proposed inductive system systems, it has been suggested to measure at least the real part of the reflected inductance (i.e. via frequency change) or to measure both the real and imaginary parts of the reflected inductance. In WO 2018/127482 for example, both the real part and imaginary part of reflected inductance $L_r$ are measured. The real part is determined by measuring the frequency of the oscillator of the resonator circuit and the imaginary part is determined by measuring loss of electromagnetic power due to absorption in the body, as well as by phase shift of the received reflected electromagnetic signal.

To measure the oscillator frequency, the required electronic circuit and microcontroller consumes a large amount of power. In case of a battery fed device, this significantly shortens the operation time between battery charges.

For example, previous proposed devices have included signal processing modules for sensing natural frequency variations. In some example, these include a circuit having a variable capacitor with a phased locked loop (PLL). In such an arrangement, the PLL artificially keeps the loop resonator frequency constant by controlling a variable capacitor in parallel with the loop capacitor. The variable capacitor control signal is thus a measure indicative of the frequency variations which would be induced in the resonator circuit absent the forcing of the PLL (i.e. variations in the natural frequency of the resonator circuit). These are in turn indicative of the real part of the additional inductance induced in the antenna as a consequence of the secondary fields emanating from the body.

In other previous examples, the resonator circuit is arranged as part of a free running oscillator circuit. The oscillator frequency is primarily influenced by the real part of the additional inductance component added to the resonator circuit by the secondary electromagnetic signals from the body. This frequency can be measured directly with a frequency counter, or an approach can be used employing the super heterodyne receiver principle, in which a second oscillator is added, and a mixer and a low pass or band pass filter are used. This is described in WO 2018/127482 for example.

Both of the above described signal sensing arrangements are complex and consume significant power.

The realization of the present invention is that an acceptable compromise can be reached between measurement versatility and precision on the one hand and power consumption on the other by measuring only the imaginary part of

reflected inductance, $L_r$, via damping variations. There had previously been a prejudice against this approach on the grounds that both real and imaginary components needed to be measured, or at least the real component, in order to ensure adequate measurement robustness. However, significant experimentation and testing by the inventors has in fact found that this is not the case, particular for simple use cases such as measurement of only one or more two particular target physiological or anatomical parameters.

Embodiments of the present invention are based on eliminating components for sensing variations in natural frequency in the resonator circuit and including means instead only for sensing damping in the resonator circuit, e.g. via variations in amplitude of the resonator oscillations, e.g. compared with the drive signal amplitude.

For example, for measuring only the respiration rate and pulse, the imaginary part of $L_r$ is sufficient. By removing high power demand and expensive electronic components for frequency measurement, and running for example a microcontroller with low clock speed to measure the imaginary part of $L_r$ at the lowest required sample rate, battery lifetime can be extended and manufacturing cost can be reduced.

A schematic block diagram of an example inductive sensing system 8 in accordance with one or more embodiments of the invention is shown in Fig. 2.

The system is for sensing electromagnetic signals returned from a body responsive to application of electromagnetic excitation signals to said body.

The system 8 comprises a resonator circuit 10 comprising: a loop antenna 12 and an electronic signal generator 14 coupled to the antenna, for driving the antenna with a drive signal to cause it to generate the electromagnetic excitation signals. The signal generator in this example is in the form of an oscillator 14 which generates the drive signal with the drive frequency. The drive frequency is preferably adjustable, for example dependent on a desired depth of measurement within the body (as will be explained further in passages to follow).

The resonator circuit 10 further includes in this example a capacitor 13 for setting or tuning a natural free space resonance frequency of the resonator circuit (i.e. natural frequency in the absence of any applied fields). The capacitor may in some examples be a variable capacitor to allow a natural free space resonance frequency to be adjusted.

The system 8 further comprises a signal sensing means 30, arranged for sensing, simultaneously with signal generation, a measure indicative of a damping exhibited in the resonator circuit relative to the drive signal supplied to the resonator circuit. The damping may refer to a damping factor of the resonator circuit. In the absence of any external magnetic fields, the resonator circuit will exhibit a natural free space damping factor, $\zeta_0$ which is associated with the degree of damping which the circuit exerts on the applied drive signal when oscillating within the resonance circuit. The secondary EM signals from the body influence the damping factor by adding a component to it which is dependent upon the properties of the object or medium in the body from which the secondary EM signals are emitted. By monitoring the variation in damping of the resonator circuit, a measurement signal carrying information related to the probed body can be derived.

The inductive sensing system is adapted to measure only said damping in the resonator circuit, and comprises no means for detecting any measure indicative of variations in frequency (e.g. a natural frequency) in the resonator circuit. Thus the sensing or measurement signal is derived solely based on detection and monitoring of the damping of the resonator circuit.

In the example of Fig. 2, the system 8 further comprises a microprocessor 32 arranged operatively coupled to the signal sensing means 30 and the resonator circuit 32. For example, the microprocessor may be configured for controlling a drive frequency of the drive signal generator 14, and/or it may be configured to perform signal processing, for example for deriving one or more physiological or anatomical parameters from the sensing or measurement signal output from the signal sensing means 30.

The signal sensing means 30 can take different forms and operate in different ways for deriving said measure representative of damping of the resonator circuit.

In some examples, the signal sensing means 30 includes at least a signal conditioning part 42 for deriving a signal indicative of the damping of the resonator circuit as a function of time, and an analog to digital converter (ADC) 44 for digitizing said derived signal. An example is schematically shown in Fig. 3.

In accordance with at least one set of embodiments, the signal sensing means 30 senses the damping based on sensing variations in amplitude of oscillations in the resonator circuit 10. For example, in some cases, the sensing of the damping may comprise sensing a measure indicative of variation in the amplitude of the resonator circuit oscillations compared with an amplitude of the drive signal applied by the oscillator 14.

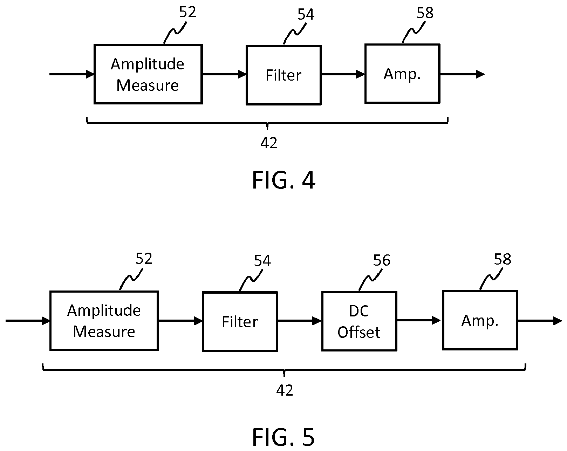

An example of such a configuration is shown schematically in Fig. 4. For illustration, Fig. 4 shows the amplitude monitoring components as comprised by the signal conditioning block 42 of the system shown in Fig. 3. This part of the signal sensing means 30 thus comprises an amplitude measurement element 52 arranged for extracting a signal indicative of amplitude of oscillating signal in the resonator circuit, a low pass or band pass filter 54 arranged for filtering the extracted amplitude signal to reduce noise, and an amplifier 58 arranged to amplify the filtered amplitude signal.

The signal sensing means may operate continuously in some examples, thereby continuously outputting as a function of time an amplitude signal representative of the detected variations in amplitude over time.

The amplitude measurement element 52 may in some examples comprise a rectifying diode. Any other means of deriving a measure of amplitude of the signal in the resonator circuit may however alternatively be used.

The filter 54 is for filtering high frequency noise, for example noise of a higher frequency than a typical upper threshold of variations in amplitude for the particular body region being probed.

The amplified amplitude signal may in some examples be output to an ADC 44 for digitization. It may then be passed in some examples to a microprocessor 32, for example for signal processing or for storage or for transmission to an external device or system or computer.

The amplifier 58 may in some examples be for increasing the power of the extracted amplitude signal to a sufficient level for it to be digitized by an ADC 44.

In some examples, the signal sensing means 30, for example a signal conditioning block 42 of such signal sensing means, may further include a DC offset adjustment element 56 between the filter 54 and the amplifier 58 for ensuring all signals passed to the amplifier have a positive DC offset (DC bias). Fig. 5 schematically depicts such an example.

The offset adjustment element 56 is thus arranged for adjusting any negative DC offset to a positive DC offset in advance of the signal passing to the amplifier 58.

In further examples, the offset adjustment element may be configured to adjust any DC offset (positive or negative) to a positive DC offset of a defined level or value. The defined level may be adjustable in some examples, or may be pre-set or pre-defined.

Many common varieties of amplifiers and ADCs are only able to handle signals having a positive DC offset voltage. Hence, the DC offset adjustment element ensures all types of amplifier (and ADC) can be used.

Furthermore, the amplified signal with DC offset should not exceed or fall below the voltage range of the ADC. Thus, adjusting the DC offset so as to be at a defined level (in advance of the signal passing to the amplifier) avoids inadvertently exceeding the maximum and minimum ADC input voltages.

Sensing variations in the amplitude of the signal oscillating in the resonator circuit 10 represents just one example approach to deriving a measure indicative of damping in the resonator circuit as a function of time. In alternative embodiments, the signal sensing means may take a different form and the damping detected in a different way.

In accordance with one or more further embodiments, deriving the measure indicative of damping of the resonator circuit may be done based on measuring a gain of the amplifying elements in the oscillator 14 circuit. The amplification gain of the oscillator is typically automatically configured to compensate any losses in the loop. Thus in this case, changes in the resonator damping may not manifest in observable changes in the amplitude of oscillations in the resonator circuit. In this case, instead the variations in the amplification gain of the oscillator 14 may be sensed and a signal indicative of these variations output as the measure indicative of variations in damping.

The oscillator 14 amplification gain can be measured by for example comparing the amplitudes of input signals to the oscillator and output signals from the oscillator. Alternatively, it may be measured by for example measuring the operation point of the active amplifier devices in the oscillator 14.

In accordance with a further set of one or more embodiments, the signal sensing means 30 may take the form of a magnetic field sensor arranged for sensing field strength of the magnetic field magnetically coupling the antenna 12 and the body being sensed. In some examples for instance it may be mounted at the location of the antenna, for example radially inside the antenna 12 of the resonator circuit 10. For example it may be arranged radially inside the antenna loop and within the plane defined by the antenna loop.

In other examples, it may be arranged at a different location to that of the antenna. For example, it may be mounted so as to be located between the plane defined by the antenna and the body to be probed. It may be arranged for example radially inside the antenna loop but axially offset from the plane defined by the antenna loop, for example offset toward the body to be probed, for example located between the antenna and the body to be probed.

In some embodiments, the system may include a frame structure or a housing to which the antenna is mounted, and wherein the magnetic field sensor is arranged also to be mounted in fixed relation with respect to the antenna.

The field strength of the magnetic field with which the loop antenna is coupled provides an indirect measure of variations in the damping factor. For example, an amplitude of magnetic field strength oscillations provide an indirect measure of consequent amplitude of the resonator oscillations, and thus of damping. Thus for example, variations downward in magnetic field strength oscillation amplitude indicates increased damping, and vice versa. Thus in some examples variation in amplitude of magnetic field strength oscillations may be used as the measure indicative of variation in damping of the resonator circuit.

According to any embodiment of the present invention, the system 8 may include a signal processing means configured, based on sensed variations in damping in the resonator circuit as a function of time, to determine one or more of heart rate and respiration rate. This may be a controller or processor in some examples. In the examples of Figs. 2-5, the microprocessor 32 performs the role of the signal processor and performs the signal processing functions.

In accordance with one or more embodiments, the system may further comprise means for setting a frequency of the drive signal based on a target measurement depth within the body.

For example, the system may include control means configured in use to set a drive frequency of said drive signal, based on a target measurement depth within the body. The control means may be provided by the microprocessor 32 for example.

As discussed above, at a fixed drive signal 14 frequency, the strength of the real and imaginary parts of the reflected inductance, $L_r$, component at the antenna 12 by secondary electromagnetic signals emitted from the body will vary as a function of the depth in the body from which the signals are being emitted (i.e. the depth of the eddy currents). In other words, for a fixed depth in the body, the strength of the real and imaginary parts will each vary between a minimum and maximum value as a function of drive signal frequency. In other words again, for every depth in the body, there is a natural optimum frequency for the drive signal for which the obtained measurement signal from the body (in the form of the imaginary part of the reflected inductance, i.e. resonator damping) will have maximum signal strength.

Thus, for a known desired measurement depth, it is possible to select the drive signal frequency so as to maximize measurement signal strength for the imaginary part (i.e. associated with damping) for that depth.

Thus, for eddy currents induced at a certain depth in the body, the oscillator 14 frequency can be chosen at a value where response in the imaginary signal is maximum.

By way of example, it might be desired to probe the heart to obtain a measure of the heart rate. In this case, a frequency for the oscillator 14 drive signal can be chosen known to have an optimum response in the imaginary reflected inductance component for the depth of the heart in the probed subject. This may thereby at the same time at least partially suppress any detected signal components received from the lungs, which are at a slightly different depth. Thus the heart can be effectively isolated.

In a further example, it might instead be desired to obtain a signal indicative of breathing rate or breathing depth. In this case, a frequency for the oscillator 14 drive signal can be chosen known to have an optimum response in the imaginary reflected inductance component for the depth of the lungs in the probed subject.

The optimal frequency choice can be the frequency where the ratio of wanted to unwanted measurements is optimal, or the frequency with the largest response of the desired physiological signal.

For example, the system may include control means configured in use to set a drive frequency of said drive signal, based on a target measurement depth within the body. The control means may for example be provided by the microcontroller 32 or microprocessor in the system of Fig. 3.

The control means may include a memory storing a lookup table, the lookup table storing a plurality of target measurement depths and associated drive signal frequencies for maximizing strength of the measurement signal from the body

The control means may be configured for receiving a user input signal indicative of the target measurement depth. The system may include a user interface for permitting input by a user of a desired measurement depth or a target anatomical region or body for sensing, and wherein the control means determines an appropriate measurement depth and, based thereon, an appropriate drive frequency for the drive signal, for maximizing acquired measurement signal strength.

As discussed, the inductive sensing system 8 according to embodiments of the present invention is configured to have means for detecting a measure of damping of the resonator circuit but not to have means for detecting variations in frequency of the resonator circuit. This saves power and complexity by avoiding components needed to derive the frequency variations.

In accordance with any embodiment of the present invention, the system may comprise a local (non-mains) power source for powering components of the system, e.g. one or more batteries. For example a battery may be arranged to supply power to the signal generation means and the signal sensing means.

In accordance with any embodiment of the present invention, the system may further comprise a housing in which the antenna 12, signal generation means 14 and signal sensing means 30 are mounted. All components of the system may be mounted inside the housing.

The system may according to one or more embodiments be a portable sensing system. For example, it may take the form of a portable inductive sensing unit, e.g. a portable probe. It may take the form of a handheld sensing device.

Examples in accordance with a further aspect of the invention also provide an inductive sensing method. The method is for sensing electromagnetic signals returned from a body responsive to application of electromagnetic excitation signals to said body based on use of a resonator circuit comprising a loop antenna.

The method comprises driving the loop antenna with a drive signal to cause it to generate the electromagnetic excitation signals

The method also comprises sensing, simultaneously with signal generation, a damping exhibited in the resonator circuit, for example relative to the drive signal supplied to the resonator circuit.

The method is characterized in that the method comprises sensing only said damping in the resonator circuit, and does not comprise detecting any measure indicative of a frequency of the resonator circuit.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (in respect of the inductive sensing system) may be applied or combined or incorporated into the present method aspect of the invention.

Embodiments of the invention provide a low cost, low current, but more functionally limited inductive sensing system. Embodiments of the system permit measurement of, by way of example, respiration rate, respiration depth and pulse.

Embodiments of the invention however permit a wide variety of different example applications. Some example applications include:

Non-invasive measurements of fluid compositions in anatomical structures, e.g. within a subject's bladder, mammary gland, or blood vessels.

Non-invasive measurements of fluid densities in tissues, e.g. lung tissue.

Application as a spot-check (hand held) device to measure for example patient respiration and pulse.

A home-use device, permitting respiration and pulse measurement for personal use.

Use for long term monitoring of respiration and pulse (enabled by using relatively low current in the resonator circuit for example).

Contactless measurements of fluid densities in e.g. baby milk bottles.

Use as a disposable device, for example based on use with a battery power source which can be disposed of with the device.

As discussed above, some embodiments make use of a control means and/or a microcontroller and/or microprocessor.

Any or each of these components may be implemented in numerous ways, with software and/or hardware, to perform the various functions required. For example a processor may be used which employs one or more microprocessors that may be programmed using software (e.g., microcode) to perform the required functions. A control means may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. An inductive sensing system (8) for sensing electromagnetic signals returned from a body responsive to application of electromagnetic excitation signals to said body, the system comprising:

   a resonator circuit (10) comprising: a loop antenna (12) and an electronic signal generator (14) coupled to the antenna, for driving the antenna with a drive signal to cause it to generate the electromagnetic excitation signals, the resonator circuit having a resonance frequency,
   a signal sensing means (30), arranged for sensing, simultaneously with signal generation, a measure indicative of a damping exhibited by the resonator circuit,
   wherein the inductive sensing system is adapted to measure only said damping in the resonator circuit, and does not detect any measure indicative of variations in frequency in the resonator circuit.

2. The system (8) as claimed in claim 1, wherein the signal sensing means (30) is adapted to monitor variation in said damping over time.

3. The system (8) as claimed in claim 1 or 2, wherein the signal sensing means (30) comprises a circuit arrangement (42, 44) electrically coupled with the resonator circuit (10).

4. The system (8) as claimed in claim 3, wherein the signal sensing means (30) is adapted to detect a measure indicative of variations in an amplitude of a measurable signal in the resonator circuit (10).

5. The system (8) as claimed in claim 4, wherein the sensing of the damping comprises sensing a measure indicative of variation in the amplitude of the measurable resonator circuit signal compared with an amplitude of the drive signal.

6. The system (8) as claimed in any of claims 3-5 wherein the circuit arrangement comprises:

an amplitude measurement element (52) arranged for extracting a signal indicative of amplitude of the resonator circuit (10) signal;
a low pass or band pass filter (54) arranged for filtering the extracted amplitude signal to reduce noise; and
an amplifier (58) arranged to amplify the filtered amplitude signal.

7. The system (8) as claimed in claim 6, wherein the circuit arrangement further comprises a DC offset adjustment element (56) between the filter (54) and the amplifier (58), arranged for adjusting any negative DC offset to a positive DC offset in advance of the signal passing the amplifier.

8. The system (8) as claimed in claim 1 wherein the signal sensing means (30) comprises a magnetic field sensor arranged in use to sense a magnetic field to which the antenna (12) of the resonator circuit (10) is exposed.

9. The system (8) as claimed in claim 8 wherein the magnetic field sensor is arranged to sense a magnetic field at a location radially inside of the loop described by the loop antenna (12).

10. The system (8) as claimed in any of claims 1-9, wherein the system includes signal processing means (32) configured, based on sensed variations in damping in the resonator circuit as a function of time, to determine one or more of heart rate and respiration rate.

11. The system (8) as claimed in any of claims 1-10, wherein the system includes control means (32) configured in use to set a drive frequency of said drive signal, based on a target measurement depth within the body.

12. The system (8) as claimed in any of claims 11, wherein the control means (32) is configured for receiving a user input signal indicative of the target measurement depth.

13. A method for sensing electromagnetic signals returned from a body responsive to application of electromagnetic excitation signals to said body based on use of a resonator circuit (10) comprising a loop antenna (12), the method comprising:

driving the loop antenna (12) with a drive signal to cause it to generate the electromagnetic excitation signals, sensing, simultaneously with signal generation, a damping exhibited in the resonator circuit, wherein the method comprises sensing only said damping in the resonator circuit, and does not comprise detecting any measure indicative of a frequency of the resonator circuit.

14. The method as claimed in claim 13, wherein sensing of the damping comprises:

detecting a measure indicative of changes in the amplitude of a measurable resonator circuit signal compared with an amplitude of the drive signal; and/or
sensing variations in a magnetic field at the location of the antenna loop.

15. The method as claimed in claim 13 or 14, further comprising setting a frequency of the drive signal based on a target measurement depth within the body.

FIG. 1

FIG. 2

FIG. 3

**FIG. 4**

**FIG. 5**

Europäisches
Patentamt

European
Patent Office

Office européen
des brevets

# EUROPEAN SEARCH REPORT

Application Number

EP 19 21 1100

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DANIEL TEICHMANN ET AL: "Respiration monitoring based on magnetic induction using a single coil", BIOMEDICAL CIRCUITS AND SYSTEMS CONFERENCE (BIOCAS), 2010 IEEE, IEEE, 3 November 2010 (2010-11-03), pages 37-40, XP031899693, DOI: 10.1109/BIOCAS.2010.5709565 ISBN: 978-1-4244-7269-7 * abstract * * section "B. System Design", paragraph 1 and 2; figure 2(b) * | 1-15 | INV. A61B5/05 A61B5/00 A61B5/024 A61B5/08 |
| X | WO 2006/111877 A1 (PHILIPS INTELLECTUAL PROPERTY [DE] ET AL.) 26 October 2006 (2006-10-26) * page 1, line 8 - line 23; figure 5 * | 1-15 | |
| A | WO 2018/077657 A1 (VIGILITECH AG [CH]) 3 May 2018 (2018-05-03) * page 19, line 7 - line 14 * | 1,11-13, 15 | TECHNICAL FIELDS SEARCHED (IPC) |
| A | WEYAND K: "AN NMR MARGINAL OSCILLATOR FOR MEASURING MAGNETIC FIELDS BELOW 50 MT", IEEE TRANSACTIONS ON INSTRUMENTATION AND MEASUREMENT, IEEE SERVICE CENTER, PISCATAWAY, NJ, US, vol. 38, no. 2, 1 April 1989 (1989-04-01), pages 410-414, XP000052869, ISSN: 0018-9456, DOI: 10.1109/19.192317 * section "Introduction", second paragraph * * passage bridging page 411 and 412; figure 2 * | 1,13 | A61B |

The present search report has been drawn up for all claims

| Place of search | Date of completion of the search | Examiner |
|---|---|---|
| The Hague | 20 February 2020 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 21 1100

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2020

| Patent document cited in search report | | Publication date | Patent family member(s) | | Publication date |
|---|---|---|---|---|---|
| WO 2006111877 | A1 | 26-10-2006 | CN | 101160093 A | 09-04-2008 |
| | | | EP | 1874185 A1 | 09-01-2008 |
| | | | JP | 2008536606 A | 11-09-2008 |
| | | | WO | 2006111877 A1 | 26-10-2006 |
| WO 2018077657 | A1 | 03-05-2018 | CN | 109963498 A | 02-07-2019 |
| | | | EP | 3531894 A1 | 04-09-2019 |
| | | | JP | 2019537733 A | 26-12-2019 |
| | | | KR | 20190067904 A | 17-06-2019 |
| | | | WO | 2018077657 A1 | 03-05-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018127482 A